# EUROPEAN PATENT APPLICATION

(11) **EP 3 760 054 A1**
(43) Date of publication of application: **06.01.2021**
(21) Application number: 19183787.1
(22) Date of filing: 02.07.2019
(51) Int. Cl.: A23L 33/135, A61K 35/74, C12Q 1/686, A61P 1/00

(54) **A METHOD OF PREPARING AN INDIVIDUAL PROBIOTIC COMPOSITION ACCORDING TO ANALYSIS OF THE MICROBIOME AND MEDICAL HISTORY FOR HUMAN OR VETERINARY USE**

(71) Applicant: Pepticus s.r.o., 616 00 Brno - Zabovresky (CZ)
(72) Inventor: ZRUST, Lukás, 602 00 Brno - Ponava (CZ)
(74) Representative: Rausch Wanischeck-Bergmann Brinkmann

(57) **Abstract**

The invention relates to a method of preparation an individually prepared probiotic composition and a food, pharmaceutical or veterinary preparation containing this composition designed to supplement the friendly microflora in the gastrointestinal tract for human or veterinary use. The composition contains a mixture of at least two different probiotic cultures mixed on the basis of analysis of the microbiome and with a view to the individual's medical history. Preparations containing a probiotic composition encompass food supplements, products for special nutrition, foodstuffs, drinks, medicines, pharmaceuticals, cosmetic and hygiene products and veterinary feeds.

## Description

### Field of the invention

The invention relates to a method for the preparation and composition of an individually prepared probiotic preparation on the basis of analysis of the microbiome and with a view to the patient's medical history designed to supplement the friendly microflora in people or animals.

### Background of the invention

The human gastrointestinal tract (GIT) is inhabited by a microbial community made up of hundreds of different species. The intestinal microflora plays a significant role in food digestion, the metabolism of endogenic and exogenic substances and the production of essential vitamins, and in increasing the immune response of the organism and preventing infections caused by pathogenic bacteria.¹

Probiotics are defined as live microbial food supplements that have a beneficial action on the host by improving the balance of the microflora in the gut.² A more recent definition from the viewpoint of human nutrition states that probiotics are live microbial food components that are beneficial to the health.³ According to the FAO/WHO (2002), probiotics are defined as live microorganisms whose administration to the host in a commensurate quantity leads to improvement to the health of the host.

In addition to proven therapeutic properties, probiotic microorganisms must also fulfil further requirements. Particular emphasis is placed on safety.⁴ This involves at least the following properties: non-pathogenic properties (including the production of toxins), resistance to stomach acids and bile, adhesion to the cells of the intestinal epithelium, the ability to colonise the gut, a proven beneficial effect on the host, and safety. Strains must be both clearly identified and also stored in an international collection of microorganisms.

Lactic acid bacteria are important probiotic microorganisms that act primarily in the human GIT. Representatives are classified by phenotypic properties, i.e. morphology, method of glucose fermentation, growth at various temperatures, production of lactic acid and fermentation of saccharides.⁵ Probiotic lactic fermentation bacteria include the genera *Lactobacillus* and *Bifidobacterium* and the species *Enterococcus faecium, Streptococcus thermophilus, Leuconostoc mesenteroides, Pediococcus acidilactici* and *Lactococcus lactis.⁵*

According to the more recent nomenclature, the genus *Bifidobacterium,* which belongs to an entirely different branch (*Actinobacteria*) than the other lactic bacteria, does not belong to the lactic fermentation bacteria group. In view of its similar physiological properties and its method of use in the food industry, and with a view to its common history, it is, however, often assigned to this group.⁶

According to current scientific studies, maintaining a suitable lactobacilli and bifidobacteria content in the gut is important to human health. A reduction to the quantity of these bacteria in the gut leads to an increased risk of metabolic disorders and other diseases.⁷ It has also been proven that maintaining an optimal ratio of the two basic bacterial phyla *Firmicutes*/*Bacteroidetes* is important to the correct function of the microbiome and energy metabolism. It has been found that an individual with a *Firmicutes*/*Bacteroidetes* ratio higher than 1 is 23% more likely to be obese.⁸ People who are extremely physically active (athletes), however, also have a higher proportion of *Firmicutes.* Bacteria of this phylum are capable of breaking down energy-rich compounds into simpler absorbable substrates, thereby increasing the energy potential of foods.⁹ This data corresponds to the data that we obtained in our research.

Both lactobacilli and bifidobacteria are represented in the microbiome of a healthy human.¹⁰ In a healthy person, bifidobacteria occur within a range of 2-14 % in the digestive tract. The amount of bifidobacteria stabilises at 3-6 %, particularly in adulthood.^{11;12} A decline in the level of bifidobacteria is very often associated with a number of diseases such as atopic dermatitis, allergies, an increased risk of oncological findings, etc. A reduced amount of bifidobacteria is characteristic of patients with IBS¹³ and patients with allergies, in which probiotic supplementation helps reduce the symptoms of allergy.¹⁴

A series of clinical studies and a number of specialist scientific publications have confirmed the positive effect of probiotic bacteria on human health.

Patent applications are focusing, at the present time, primarily on methods of analysing the microbiome. An example is patent RU2015146401, in which the authors describe a method of microbiome culture analysis with the use of various culture media. Document CN109115570 concerns a method of fully automated detection of microbiome dysbiosis without considering probiotic supplementation. EP2985350 describes a modification of a method of microbiome analysis by means of the addition of a defined amount of a standard, which enables more precise quantification of the individual bacterial constituents of the microbiome. Document US2017268046 concerns a method of collection and stabilisation of a stool sample and its subsequent analysis according to the content of nucleic acid sequences. In document RU2666608, the authors describe a way of treating foot mycoses in which determination of the content of lactobacilli, bifidobacteria and *E. coli* comprises part of the process of selecting an antifungal treatment. Document US2017356029 considers microbiome typing in relation to autism, though with no connection of any kind to specific probiotic supplementation.

Certain patent applications such as, for example, RU2177152 and RU2412989 focus on screening lactobacilli and bifidobacteria with special emphasis on their antagonistic effect towards selected bacterial pathogens. Document RU2428468 is based on the selection of probiotic bacteria capable of inhibiting pathogenic bacteria isolated from the patient's gut. The Russian patent application RU2002125639 concerns the preparation of suppositories with the use of two bacterial phyla without considering the composition of the microbiome.

Other documents consider the preparation of a probiotic preparation containing bifidobacteria, though again with no consideration of microbiome analysis. Patent application RU2126043 concerns a method of preserving and isolating strains from stool samples from a healthy human and their subsequent multiplication and administration to the donor at a time of illness. This involves, in essence, the preparation of autogenic probiotics. Modification of the composition of the probiotic preparation in dependence on the composition of the microbiome of the recipient of the probiotics is again not considered here. Korean patent application KR20190025180 focuses on analysis of the microbiome of animals with subsequent medical consultation, though not on proposal of the composition of probiotics. Document TW201732724 is concerned with a method of developing a probiotic product and a system for its preparation, this not on an individual basis but targeting wider groups of patients with a view to a diagnosis and the regional specificity of the microbiome.

Today, all probiotics come in the form of dietary supplements, medical pharmaceuticals, drugs, veterinary dietary preparations, foodstuffs or special-purpose foodstuffs conceived on a universal principle that enables the large-volume production of such preparations. Such probiotic preparations do not respect the individual needs of their users. While it is true that probiotics specialised for various indications are available on the market, even these do not respect an individualised approach.

Characterisation of the intestinal microbiome is currently performed largely by sequencing analysis¹⁷, which involves the quantification of bacteria-specific 16S rRNA gene sequences. The Real Time PCR method⁸ is another widely used method for determining the relative amount of bacterial DNA of selected bacterial species, genera or phyla. Standard culturing¹⁵, in which determination of the quantity of bacterial cells, with the possibility of subsequent identification of the biochemical profile¹⁶, is performed by culturing a diluted sample on selective media, is a conservative method of characterising the intestinal microbiome.

Today, all probiotics come in the form of dietary supplements, medical pharmaceuticals, drugs, veterinary dietary preparations, foodstuffs or special-purpose foodstuffs conceived on a universal principle that enables the large-volume production of such preparations. Such probiotic preparations do not respect the individual needs of their users with a view to the actual state or composition of the intestinal microbiome. While it is true that probiotics specialised for various indications are available on the market, even these do not respect an individualised approach.

### Literature

¹ GIBSON, G. R.; ROBERFROID, M. B. Dietary Modulation of the Human Colonic Microbiota: Introducing the Concept of Prebiotics. 1995. 1401-12.
² FULLER, R. Probiotics in man and animals. J Appl Bacteriol 66: 365-378. 1989. 365-78.
³ SALMINEN, S. et al. Demonstration of safety of probiotics - a review. 1998.
⁴ FRIC, P. Probiotics and prebiotics - Renaissanceof a therapeutic principle. 2007. 237-270.
⁵ HOLZAPFEL, W. et al. Taxonomy and important features of probiotic microorganisms in food and nutrition. 2001. 365S-373S.
⁶ BORRIELLO, S. P. et al. Safety of probiotics that contain lactobacilli or bifidobacteria. Clin Infect Dis, v. 36, n. 6, p. 775-80, Mar 2003. ISSN 1537-6591. Available at:https://www.ncbi.nlm.nih.gov/pubmed/12627362.
⁷ CROVESY, L. et al. Effect of Lactobacillus on body weight and body fat in overweight subjects: a systematic review of randomized controlled clinical trials. Int J Obes (Lond), v. 41, n. 11, p. 1607-1614, 11 2017. ISSN 1476-5497. Available at: https://www.ncbi.nlm.nih.gov/pubmed/28792488.
⁸ KOLIADA, A. et al. Association between body mass index and Firmicutes/Bacteroidetes ratio in an adult Ukrainian population. BMC Microbiol, v. 17, n. 1, p. 120, 05 2017. ISSN 1471-2180. Available at: https://www.ncbi.nlm.nih.gov/pubmed/28532414.
⁹ MACH, N.; FUSTER-BOTELLA, D. Endurance exercise and gut microbiota: A review. J Sport Health Sci, v. 6, n. 2, p. 179-197, Jun 2017. ISSN 2213-2961. Available at: https://www.ncbi.nlm.nih.gov/pubmed/30356594.
¹⁰ SGHIR, A. et al. Quantification of bacterial groups within human fecal flora by oligonucleotide probe hybridization. Appl Environ Microbiol, v. 66, n. 5, p. 2263-6, May 2000. ISSN 0099-2240. Available at: https://www.ncbi.nlm.nih.gov/pubmed/10788414.
¹¹ HOPKINS, M.; SHARP, R.; MACFARLANE, G. Age and disease related changes in intestinal bacterial populations assessed by cell culture, 16S rRNA abundance, and community cellular fatty acid profiles. 2001. 198-205.
¹² SATOKARI, R. et al. Molecular Approaches for the Detection and Identification of Bifidobacteria and Lactobacilli in the Human Gastrointestinal Tract. 2003. 572-84.
¹³ TAVERNITI, V.; GUGLIELMETTI, S. Methodological issues in the study of intestinal microbiota in irritable bowel syndrome. 2014. 8821-8836.
¹⁴ AKAY, H. et al. The relationship between bifidobacteria and allergic asthma and/or allergic dermatitis: A prospective study of 0-3 years-old children in Turkey. 2014.
¹⁵ DELGADO, S.; SUAREZ, A.; MAYO, B. Identification of dominant bacteria in feces and colonic mucosa from healthy Spanish adults by culturing and by 16S rDNA sequence analysis. Dig Dis Sci, v. 51, n. 4, p. 744-51, Apr 2006. ISSN 0163-2116. Available at: https://www.ncbi.nlm.nih.gov/pubmed/16614998.
¹⁶ TALAIEKHOZANI, A.; ALAEE, S.; MOHANADOSS, P. Guidelines for quick application of biochemical tests to identify unknown bacteria. 2015. 65-82.
¹⁷ DI SEGNI, A. et al. Guided Protocol for Fecal Microbial Characterization by 16S rRNA-Amplicon Sequencing. J Vis Exp, n. 133, 03 2018. ISSN 1940-087X. Available at: https://www.ncbi.nlm.nih.gov/pubmed/29608151.
¹⁸ THOMAS, D. J. et al. Lactobacillus rhamnosus HN001 attenuates allergy development in a pig model. PLoS One, v. 6, n. 2, p. e16577, Feb 2011. ISSN 1932-6203. Available at: https://www.ncbi.nlm.nih.gov/pubmed/21386995.
¹⁹ KALLIOMAKI, M. et al. Probiotics in primary prevention of atopic disease: a randomised placebo-controlled trial. Lancet, v. 357, n. 9262, p. 1076-9, Apr 2001. ISSN 0140-6736. Available at: https://www.ncbi.nlm.nih.gov/pubmed/11297958.
²⁰ TOSCANO, M. et al. Effect of. World J Gastroenterol, v. 23, n. 15, p. 2696-2704, Apr 2017. ISSN 2219-2840. Available at: https://www.ncbi.nlm.nih.gov/pubmed/28487606.
²¹ PAINEAU, D. et al. Effects of seven potential probiotic strains on specific immune responses in healthy adults: a double-blind, randomized, controlled trial. FEMS Immunol Med Microbiol, v. 53, n. 1, p. 107-13, Jun 2008. ISSN 0928-8244. Available at: https://www.ncbi.nlm.nih.gov/pubmed/18422632.
²² LEYER, G. et al. Probiotic Effects on Cold and Influenza-Like Symptom Incidence and Duration in Children. 2009. e172-9.
²³ FORSSTEN, S. et al. Influence of a probiotic mixture on antibiotic induced microbiota disturbances. 2014. 11878-85.
²⁴ ENGELBREKTSON, A. et al. Probiotics to minimize the disruption of faecal microbiota in healthy subjects undergoing antibiotic therapy. J Med Microbiol, v. 58, n. Pt 5, p. 663-70, May 2009. ISSN 0022-2615. Available at: https://www.ncbi.nlm.nih.gov/pubmed/19369530.
²⁵ OUWEHAND, A. C. et al. Probiotics reduce symptoms of antibiotic use in a hospital setting: a randomized dose response study. Vaccine, v. 32, n. 4, p. 458-63, Jan 2014. ISSN 1873-2518. Available at: https://www.ncbi.nlm.nih.gov/pubmed/24291194.
²⁶ GOMES, A. C. et al. The additional effects of a probiotic mix on abdominal adiposity and antioxidant status: A double-blind, randomized trial. Obesity (Silver Spring), v. 25, n. 1, p. 30-38, 01 2017. ISSN 1930-739X. Available at: https://www.ncbi.nlm.nih.gov/pubmed/28008750.
²⁷ ANDREASEN, A. et al. Effects of Lactobacillus acidophilus NCFM on insulin sensitivity and the systemic inflammatory response in human subjects. 2010. 1831-8.
²⁸ RINGEL-KULKA, T. et al. Probiotic bacteria Lactobacillus acidophilus NCFM and Bifidobacterium lactis Bi-07 versus placebo for the symptoms of bloating in patients with functional bowel disorders:adouble-blind study. 2011. 518-25.
²⁹ SCHMIDT, E. et al. Antigen-presenting cells exposed to Lactobacillus acidophilus NCFM, Bifidobacterium bifidum BI-98, and BI-504 reduce regulatory T cell activity. 2010. 390-400.
³⁰ LAMIKI, P. et al. Probiotics in Diverticular Disease of the Colon: an Open Label Study. 2010. 31-6.
³¹ RINGEL-KULKA, T. et al. Lactobacillus acidophilus NCFM affects colonic mucosal opioid receptor expression in patients with functional abdominal pain - a randomised clinical study. 2014.
³² WICKENS, K. et al. Effects of Lactobacillus rhamnosus HN001 in early life on the cumulative prevalence of allergic disease to 1 lyears. Pediatr Allergy Immunol, v. 29, n. 8, p. 808-814, 12 2018. ISSN 1399-3038. Available at: https://www.ncbi.nlm.nih.gov/pubmed/30430649.
³³ CACERES, P. et al. Effects of Lactobacillus rhamnosus HN001 on acute respiratory infections and intestinal secretory IgA in children. 2010. 353-362.
³⁴ KONSTANTINOV, S. R. et al. S layer protein A of Lactobacillus acidophilus NCFM regulates immature dendritic cell and T cell functions.2008.
³⁵ SLYKERMAN, R. F. et al. Effect of Lactobacillus rhamnosus HN001 in Pregnancy on Postpartum Symptoms of Depression and Anxiety: A Randomised Double-blind Placebo-controlled Trial. EBioMedicine, v. 24, p. 159-165, Oct 2017. ISSN 2352-3964. Available at: https://www.ncbi.nlm.nih.gov/pubmed/28943228.
³⁶ WICKENS, K. et al. Early pregnancy probiotic supplementation with Lactobacillus rhamnosus HN001 may reduce the prevalence of gestational diabetes mellitus: a randomised controlled trial. 2017. 1-10.
³⁷ JANG, S. E. et al. Lactobacillus rhamnosus HN001 and Lactobacillus acidophilus La-14 Attenuate Gardnerella vaginalis-Infected Bacterial Vaginosis in Mice. Nutrients, v. 9, n. 6, May 2017. ISSN 2072-6643. Available at: https://www.ncbi.nlm.nih.gov/pubmed/28545241.
³⁸ CORREA, N. B.et al. Treatment of Acute Diarrhea With Saccharomyces Boulardii in Infants. 2011. 497-501.

### Summary of the invention

The invention provides a solution to the shortcomings of the state of the art, particularly in the area of individualised production of probiotic preparations. This is based on a combination of two factors - an intestinal microbiome analysis of the individual and the individual's medical history - on the basis of which a specific formulation of selected probiotic cultures for a resultant probiotic composition is prepared.

The subject of the invention is a method of preparing an individual probiotic composition for human or veterinary use according to an individual's medical history based on analysis of the individual intestinal microbiome, taking in analysis of the intestinal microbiome of the individual by means of analysis of a stool sample for quantitative determination of bacteria of the genera *Lactobacillus* and *Bifidobacterium* and determination of their ratio and/or determination of the ratio of the bacterial phyla *Firmicutes* and *Bacteroidetes* in the sample. The proportions of individual bacterial phyla in the total intestinal microbiome of the individual can also be determined. The medical history of the individual, i.e. records of diseases and all medical disorders, is obtained at the same time.

The next step is preparation of the constituent of individual probiotic cultures for a probiotic composition according to the results of analysis of the intestinal microbiome with the use of probiotic cultures with proven health benefits for at least one of the disorders stated in the medical history of the individual according to at least one source in the literature and/or the result of a clinical study. The following step involves ensuring that the individually prepared probiotic composition contains those species or genera of bacteria that are, according to the results of microbiome analysis, present in a reduced quantity or lacking in the gut in comparison with the representation of bacterial cultures in the microbiome of a healthy person. Probiotic bacteria that are clinically tested for indications obtained from the individual's medical history are selected on a preferential basis to supplement the lacking genera of bacteria. This is a unique combination - supplementation of bacteria from the group of microorganisms whose quantity is reduced or that are entirely missing from the patient's microbiome with the preferential selection of strains clinically tested for the selected anamnesis. An excipient is finally added to the prepared constituent of individual probiotic cultures for the probiotic composition.

Analysis of the microbiome in the stool sample is, according to the preferred embodiment, performed by means of culturing with the possibility of subsequent identification of the biochemical profile and/or Real Time PCR and/or sequencing of the stool sample, or by any combination of the given methods.

The subject of the invention is also the probiotic composition prepared by the method of the invention, wherein the constituent of individual probiotic cultures contains a mixture of at least two different probiotic cultures and an excipient.

The constituent of individual probiotic cultures preferentially contains bacterial genera selected from the group *Lactobacillus, Bifidobacterium, Streptococcus, Bacillus, Saccharomyces, Enterococcus, Lactococcus, Escherichia, Akkermansia, Faecalibacterium, Eubacterium, Leuconostoc* and *Pediococcus* or mixtures of them.

The probiotic composition according to the invention contains bacterial cultures at a quantity of 1 x 10³ to 1 x 10¹⁴ CFU (colony forming units) per 1 g of total weight of the probiotic composition, with at least one probiotic culture at a quantity of at least 1 x 10⁵ to 1 x 10¹⁴ CFU per 1g of total weight of the probiotic composition, and preferentially 50 x 10⁹ CFU to 200 x 10⁹ CFU per 1 g of total weight of the probiotic composition.

According to a preferred embodiment, the probiotic composition contains 1-34% bifidobacteria and 66-99% lactobacilli, preferentially 25% bifidobacteria and 75% lactobacilli of the total number of probiotic cultures of the constituent of individual probiotic cultures in the probiotic composition if the ratio of lactobacilli and bifidobacteria in the stool sample is less than 0.099.

If the ratio of lactobacilli and bifidobacteria in the stool sample falls within a range of 0.1 to 0.799, the probiotic composition contains 2-49% bifidobacteria and 51-98% lactobacilli, preferentially 35% bifidobacteria and 65% lactobacilli of the total number of probiotic cultures of the constituent of individual probiotic cultures in the probiotic composition.

If the ratio of lactobacilli and bifidobacteria in the stool sample falls within a range of 0.8 to 1.099, the probiotic composition contains 20-80% bifidobacteria and 20-80% lactobacilli, preferentially 50% bifidobacteria and 50% lactobacilli of the total number of probiotic cultures of the constituent of individual probiotic cultures in the probiotic composition.

If the ratio of lactobacilli and bifidobacteria in the stool sample is higher than 1.1, the probiotic composition contains 20-99% bifidobacteria and 1-80% lactobacilli, preferentially 75% bifidobacteria and 25% lactobacilli of the total number of probiotic cultures of the constituent of individual probiotic cultures in the probiotic composition.

If the ratio of the bacterial phyla *Firmicutes* and *Bacteroidetes* in the stool sample is higher than 1, the probiotic composition contains probiotic bacteria of the genus *Bifidobacterium.* If the patient also has a BMI (Body Mass Index) higher than 25, probiotic bacteria that have, according to at least one source in the literature and/or the result of a clinical study, a confirmed effect against obesity, metabolic disorders and diabetes, such as the bacterium *Bifidobacterium lactis* Bl-04, are used preferentially for the preparation of the probiotic composition.

If the ratio of the bacterial phyla *Firmicutes* and *Bacteroidetesin* the stool sample is lower than 0.8, the probiotic composition contains probiotic bacteria of the genus *Lactobacillus* at an amount of 51-100% of the total number of probiotic cultures of the constituent of individual probiotic cultures in the probiotic composition.

If the ratio of the bacterial phyla *Firmicutes* and *Bacteroidetes* in the stool sample is higher than 0.8 and lower than 1, the probiotic composition contains probiotic bacteria of the genus *Lactobacillus* at an amount of 1-99% and bacteria of the genus *Bifidobacteria* at an amount of 99-1 % of the total number of probiotic cultures of the constituent of individual probiotic cultures in the probiotic composition.

The excipient of the probiotic composition may be advantageously selected from a group containing inulin, dextrin, maltodextrin, maize starch, potato starch, fructooligosaccharides, galactooligosaccharides, pectin, guar gum, xanthan gum, carrageenan, alginate, fruit extracts, vegetable extracts, cereal extracts, legume extracts, lyophilised fruit, lyophilised vegetables, vitamins, minerals, plant and herb extracts and mixtures of them and other physiologically beneficial substances with a prebiotic potential at a maximum quantity of up to 99.9% by weight.

The subject of the invention is also a food, pharmaceutical or veterinary preparation containing a probiotic composition in accordance with the invention, which may advantageously take the form of a tablet, capsule, powder, gel, suppository, sweet, yoghurt, kefir, solution or granulate. Preparations containing a probiotic composition encompass food supplements, products for special nutrition, foodstuffs, drinks, medicines, pharmaceuticals, cosmetic and hygiene products and veterinary feeds.

The invention is explained further with the use of application examples, which do not, however, restrict in any way other possible applications to the extent of the patent claims.

### Brief description of drawings

Figure 1: Content of lactobacilli in % of the total amount of bacterial cultures in the stool sample before use of the probiotic preparation and after 3 months of use of the preparation according to example 1.
Figure 2: Content of bifidobacteria in % of the total amount of bacterial cultures in the stool sample before use of the probiotic preparation and after 3 months of use of the preparation according to example 1.
Figure 3: Analysis of the representation of bacterial strains in the stool sample before probiotic supplementation according to example 2.
Figure 4: Results of analysis of the stool sample for the quantity of bacterial species before use and after use of the probiotic preparation according to example 2.
Figure 5: Content of lactobacilli in % of the total amount of bacterial cultures in the stool sample before use of the probiotic preparation and after 3 months of use of the preparation according to example 2.
Figure 6: Content of bifidobacteria in % of the total amount of bacterial cultures in the stool sample before use of the probiotic preparation and after 3 months of use of the preparation according to example 2.
Figure 7: Analysis of the representation of bacterial strains in the stool sample before probiotic supplementation according to example 4.
Figure 8: Analysis of the representation of bacterial strains in the stool sample before probiotic supplementation according to example 6.

### Examples of the invention embodiments

### Example 1

The ratio of lactobacilli and bifidobacteria in the stool sample was determined by molecular biological sequence analysis and subsequently by Real Time PCR at a value of 0.084. It was decided on the basis of this ratio that 25% of the daily dose of probiotics (35 x 10⁹ CFU) would be comprised of bifidobacteria and 75 % of lactobacilli. The client, a 45-year-old woman, showed allergies and dermatitis in her medical history, for which reason preferential use was made of probiotic bacteria that are shown by clinical studies or another publication output to have a therapeutic effect on allergies¹⁸ and dermatitis¹⁹ supplemented by other probiotic species to increase the overall bacterial diversity in the intestine. A fructooligosaccharide from chicory - inulin - was used as an auxiliary prebiotic substance. A mixture of commercially available probiotic cultures as given in Table 1 was homogenised in a dry state in a laboratory homogeniser for 10 minutes. Filling in capsules took place with the use of a manual capsule filler in a laboratory with regulated air humidity to 35% and temperature to 22°C.

The resultant composition of a weight of 450 mg, representing the daily dose, was filled in size 0 gelatine capsules with the use of a manual capsule filler. The resultant content of the individual probiotic composition was as follows:

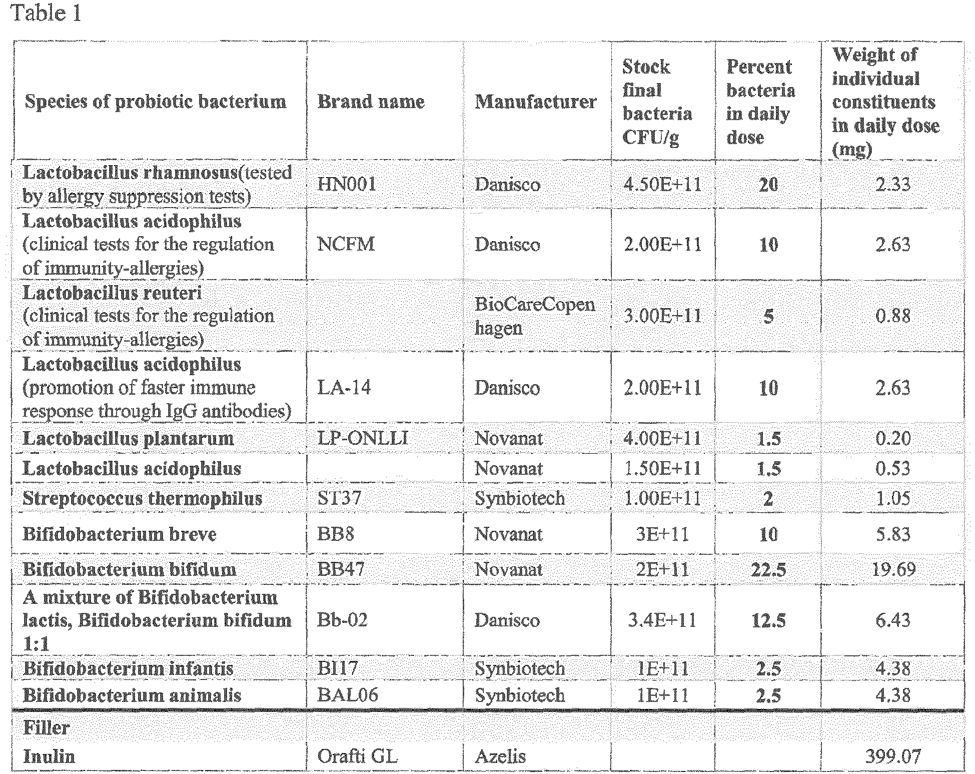

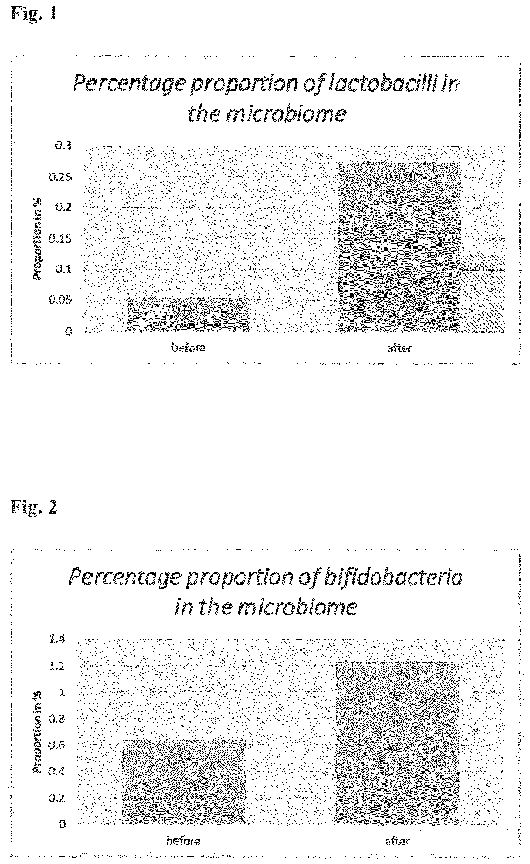

A stool sample was again analysed after 3 months of use of the probiotic preparation and the content of lactobacilli and bifidobacteria determined in % of the total amount of bacterial cultures in the sample (Figures 1 and 2).

### Example 2

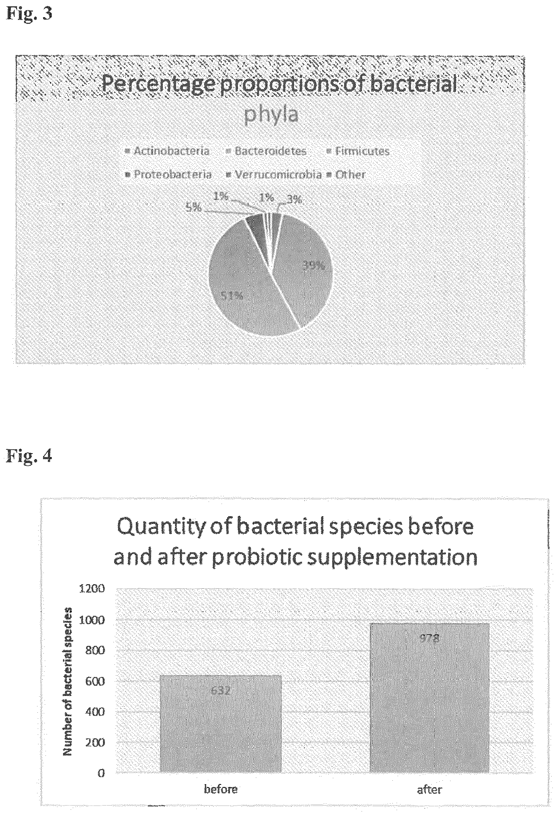

The ratio of lactobacilli and bifidobacteria in the stool sample was determined by molecular biological analysis with the use of Real Time PCR at a value of 0.019. The ratio of the bacterial phyla *BacteroideteslFirmicutes* was determined as a value of 0.75 by sequence analysis and the proportions and quantity of bacterial phyla (Figure3) and the quantity of bacterial species (Figure 4) in the stool sample were also determined. The proportion of probiotic cultures in the resultant formulation was proposed as 25% bifidobacteria and 75 % lactobacilli in the daily dose of probiotic bacteria. In view of the young age of the client (9 years) the daily dose of probiotics was set at 20 x 10⁹ CFU. No illnesses were stated in the client's medical history, for which reason probiotic species of bacteria targeting increased bacterial diversity in the intestine with an emphasis on optimising digestion²⁰ and reinforcing overall immunity^{21;22} were used in the probiotic composition - *Bifidobacterium lactis Bi-07.* A fructooligosaccharide from chicory - inulin - was used as an auxiliary prebiotic substance.

The resultant content of the probiotic composition of the powder mix is given in Table 2. The composition of a weight of 1,000 mg, corresponding to the daily dose, was filled in single-use bags.

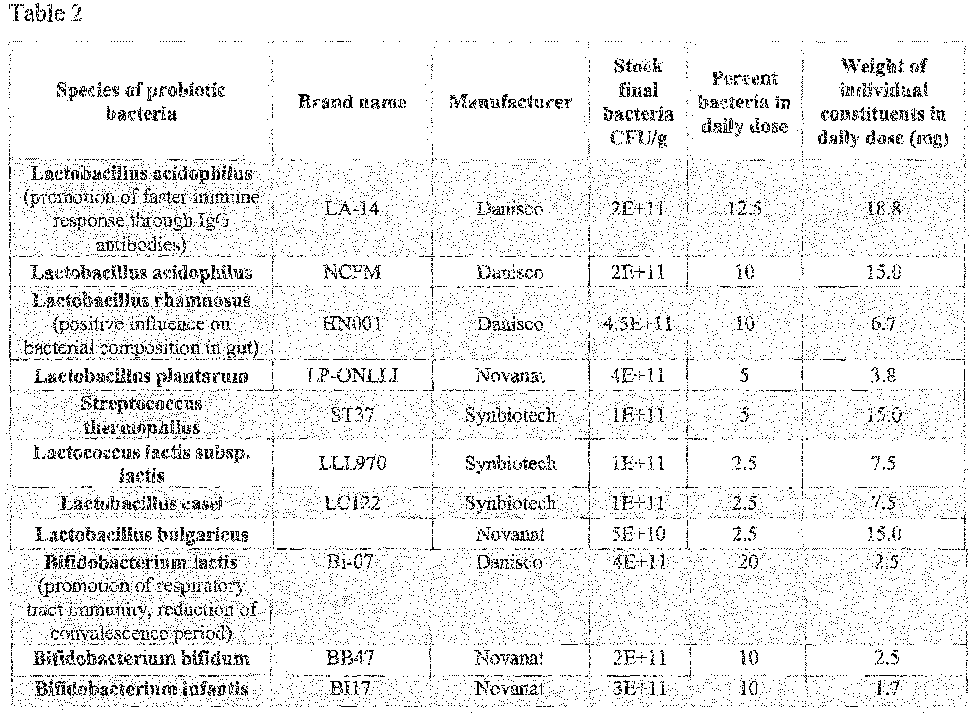

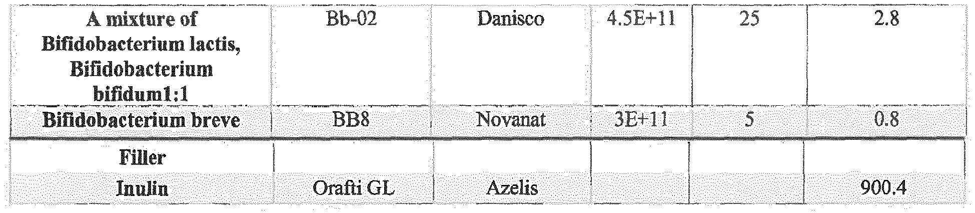

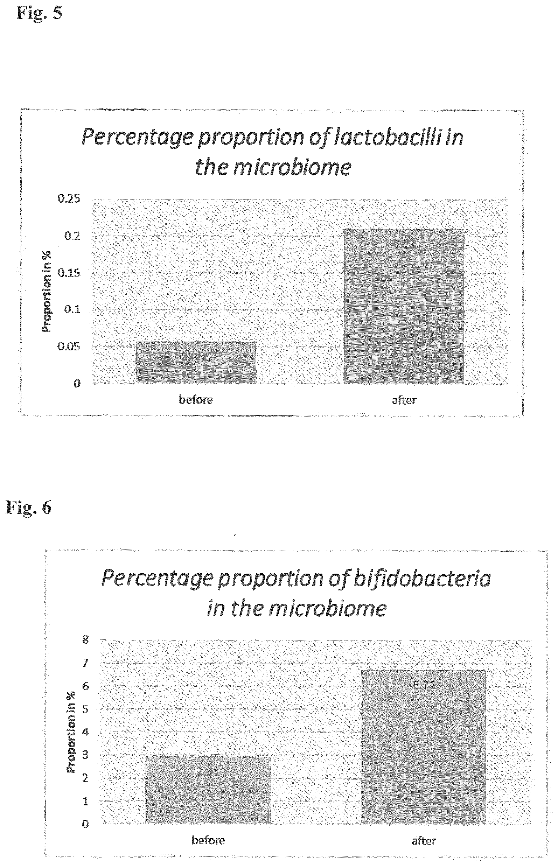

A stool sample was again analysed after 3 months of use of the probiotic preparation and the quantity of bacterial species (Figure 4) and the content of lactobacilli and bifidobacteria in % of the total amount of bacterial cultures in the sample (Figures 5 and 6) were determined.

### Example 3

The ratio of lactobacilli and bifidobacteria in the stool sample was determined by molecular biological sequence analysis at a value of 0.93 and the quantity of bacterial species in the sample was also determined. The proportion of probiotic bacteria in the resultant probiotic composition was stipulated as 50 % bifidobacteria and 50 % lactobacilli in the daily dose of probiotic bacteria. Comprehensive molecular biological analysis pointed to considerably reduced diversity of the client's intestinal microbiome. The reason for this may have been the recent intensive antibiotic treatment of *H. pylori* given in the client's medical history. The client further stated frequent diarrhoeal conditions. For these reasons, the daily dose was set at 45 x 10⁹ CFU fortified primarily by bacterial species capable of reducing the negative effect of antibiotics on the bacterial microflora and alleviating diarrhoeal conditions.^{21;14;21} In view of these diarrhoeal conditions a mixture of hydroxypropyl methylcellulose to an amount of 10 % of the total content of the capsule and SiO₂ at an amount of 1 % of the total content of the tablet, promoting the adhesion of probiotic bacteria to the intestinal epithelium, was used as an auxiliary prebiotic substance. The mixture was supplemented by magnesium stearate at 0.5 % of the total tablet content and by lyophilised banana.

The contents of the resultant composition correspond to the data given in Table 3A. The composition, of a weight of 450 mg adjusted to oval tablets, corresponded to the daily dose of the preparation.

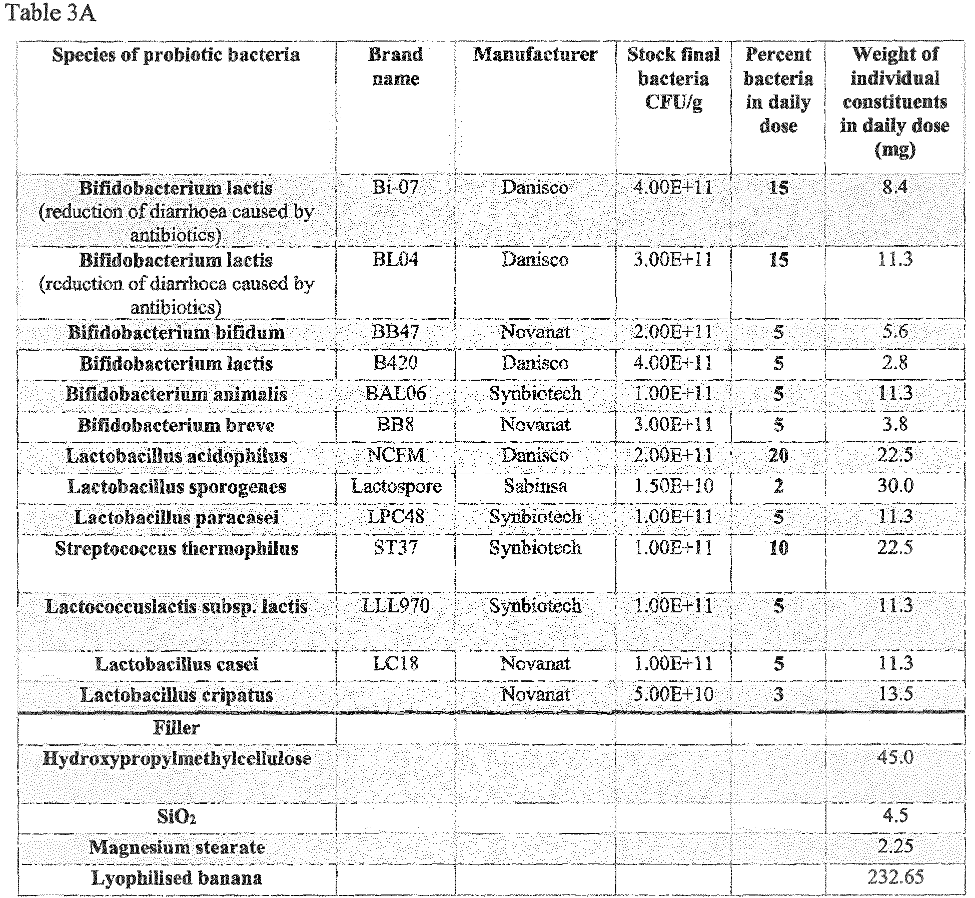

A stool sample was again analysed after 3 months of use of the probiotic preparation and the content of lactobacilli and bifidobacteria in % of the total amount of bacterial cultures in the sample and the total number of bacterial species in the sample were determined (Table 3B).

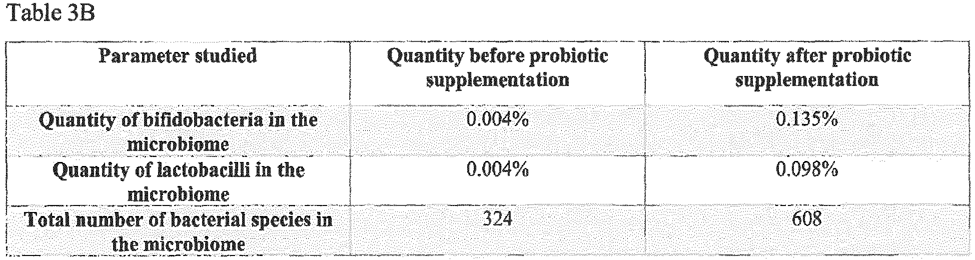

### Example 4

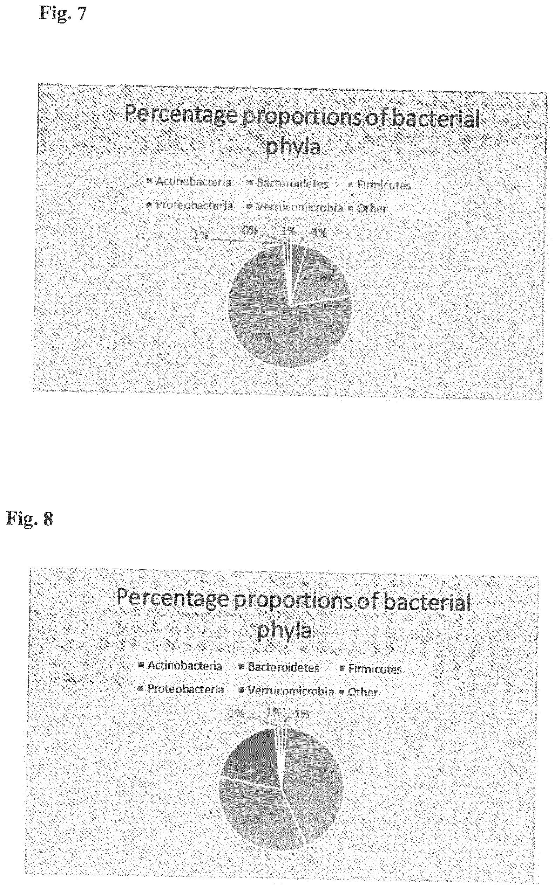

The ratio of lactobacilli and bifidobacteria in the stool sample was determined by molecular biological analysis at a value of 1.4. It was decided on the basis of this ratio that 75% of the daily dose of probiotics (35 x 10⁹CFU) would be formed of bifidobacteria and 25 % of lactobacilli. Molecular biological analysis revealed a significantly increased ratio (4.2) of bacteria of the phyla *FirmicuteslBacteroidetes.* This trend is characteristic of obese patients, which corresponds to the increased BMI (Body Mass Index, 34.2) of the (female) client. The proportion and quantity of bacterial strains (Figure 7), the overall number of bacterial species in the sample and the level of glucose in the blood on an empty stomach were determined. The client stated diabetes II in her medical history, for which reason probiotic bacteria that have a clinically verified effect against obesity²⁶ and that have a beneficial effect on insulin sensitivity²⁷ were used on a preferential basis. The resistant gluten-free wheat starch Nutriose FB06 at an amount of 40% of the total content of the capsule, supplemented by lyophilised carrot, was used as an auxiliary prebiotic substance.

The resultant composition of a weight of 550 mg, adjusted for size 00 HPMC capsules corresponding to the daily dose of the preparation, had the composition given in Table 4A.

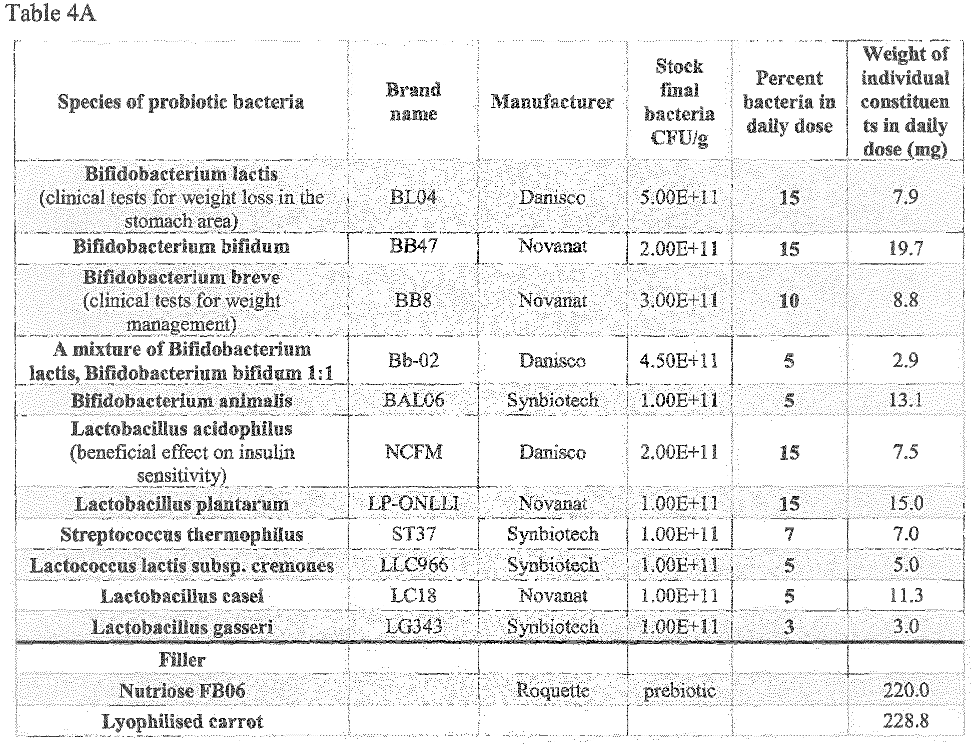

A stool sample was again analysed after 3 months of use of the probiotic preparation and the content of lactobacilli and bifidobacteria in % of the total amount of bacterial cultures in the sample, the total number of bacterial species in the sample and the level of glucose in the blood on an empty stomach were determined (Table 4B).

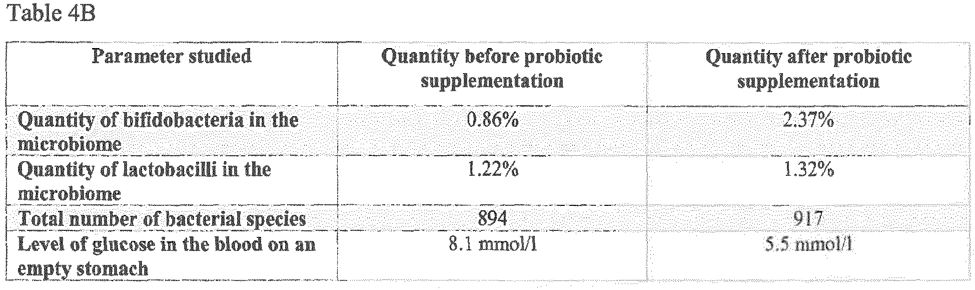

### Example 5

The ratio of lactobacilli and bifidobacteria in the stool sample was determined by molecular biological analysis at a value of 0.237. It was decided on the basis of this ratio that 35 % of the daily dose of probiotics (35 x 10⁹ CFU) would be formed of bifidobacteria and 65 % of lactobacilli. The total number of bacterial species in the sample and the level of cholesterol in the blood were also determined. The client, a 39-year-old man, stated persistent constipation, bloating and meteorism in his medical history, for which reason probiotic bacteria that have been proven by clinical studies or other published output to have a therapeutic effect against constipation, supplemented by other probiotic species to reduce digestive discomfort²⁸ and increase overall bacterial diversity in the gut²¹, were used on a preferential basis. Lactulose, which acts against constipation, was used to an amount of 10% of the total content of the capsule and was supplemented by an auxiliary prebiotic substance - maltodextrin.

The resultant probiotic composition of a weight of 500 mg, corresponding to the daily dose, designed for the production of a solution or to be dissolved in a lukewarm drink, had the composition given in Table 5A.

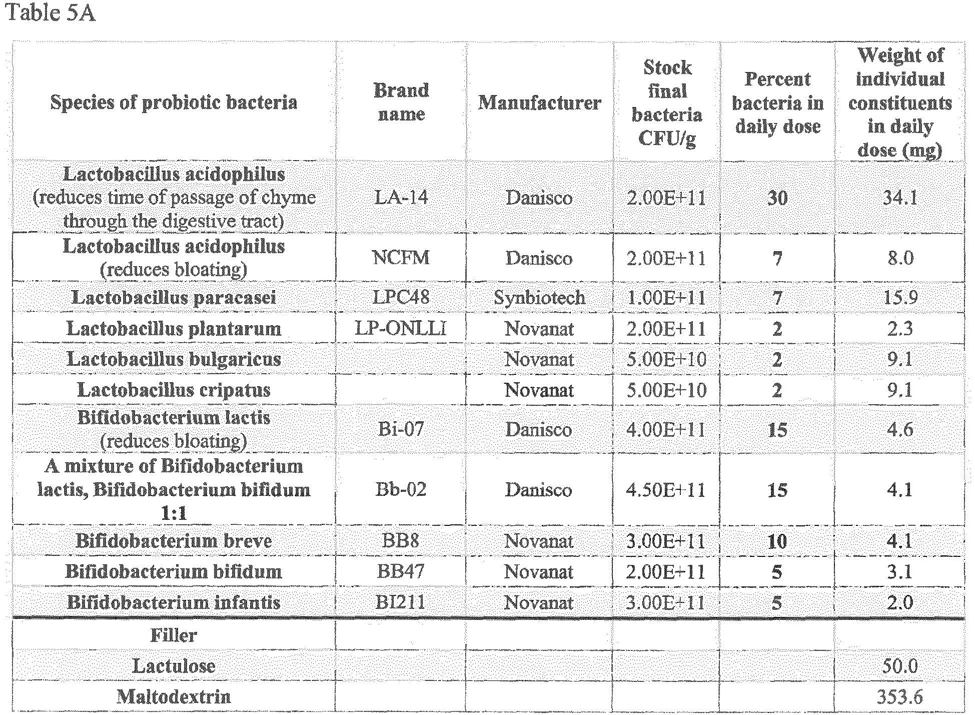

A stool sample was again analysed after 3 months of use of the probiotic preparation and the content of lactobacilli and bifidobacteria in % of the total amount of bacterial cultures in the sample, the total number of bacterial species in the sample and the level of cholesterol in the blood were determined (Table 5B).

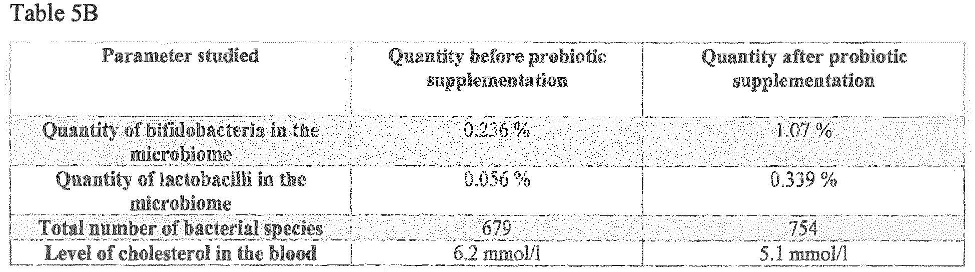

### Example 6

The ratio of lactobacilli and bifidobacteria in the stool sample was determined by molecular biological sequence analysis at a value of 0.421. The proportion of probiotic bacteria in the resultant probiotic composition was stipulated as 35 % bifidobacteria and 65 % lactobacilli. The proportion and quantity of bacterial stains (Figure 8) and the total number of bacterial species in the sample were determined. The *Firmicutes*/*Bacteroidetes* ratio was determined as 1.12 and the client's medical history also gave a BMI of 22. Comprehensive molecular biological analysis pointed to a considerably reduced diversity in the client's intestinal microbiome. The client's medical history also stated diagnosed Crohn's disease accompanied by strong digestive discomfort and diarrhoea with defecation 5-6 times a day. The daily dose was, for these reasons, set at 45 x 10⁹ CFU. Probiotic bacteria proven by clinical studies or other published output to have a therapeutic effect in relation to non-specific intestinal inflammations^{29;30}, accompanied by other probiotic species with the aim of reducing digestive discomfort^{28;31} and increasing overall bacterial diversity in the gut²¹, were used on a preferential basis in the probiotic composition. A mixture of Gellan gum at a quantity of 10 % of the total content of the capsule and SiO₂ at an amount of 1 % of the total content of the capsule, promoting the adhesion of probiotic bacteria to the intestinal epithelium, was used in view of the client's diarrhoeal conditions as an auxiliary prebiotic substance. The mixture was supplemented with lyophilised banana.

The resultant probiotic composition of a weight of 450 mg, adjusted for size 0 enterosolvent HPMC capsules and corresponding to the daily dose, had the composition given in Table 6A.

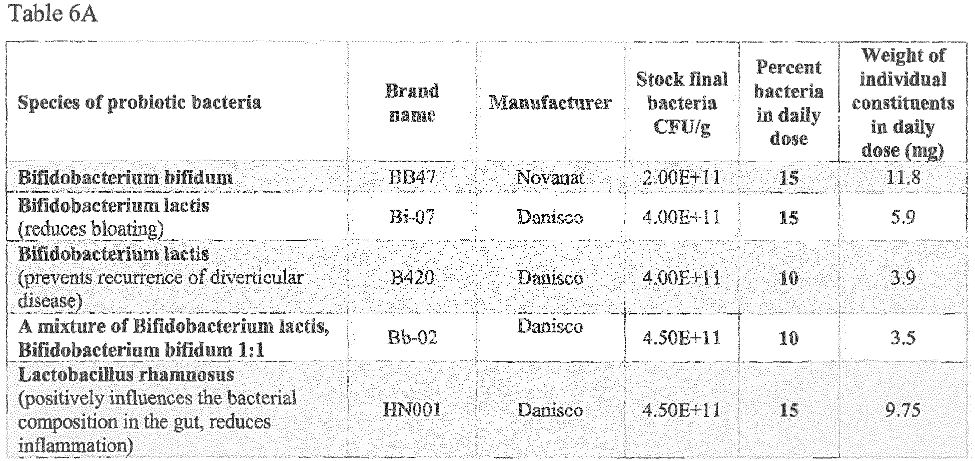

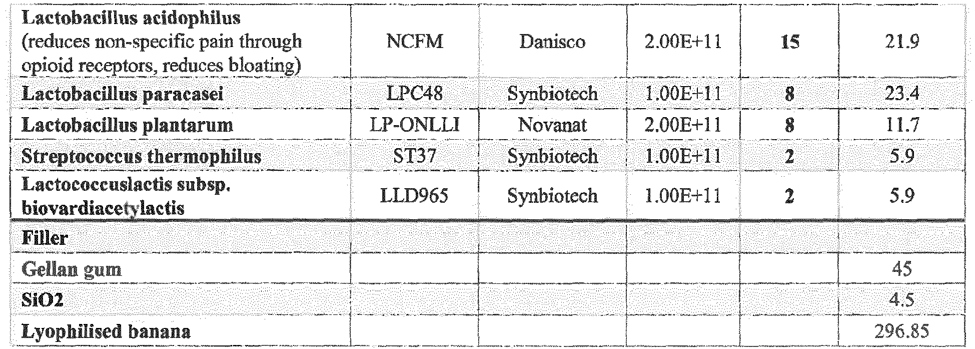

A stool sample was again analysed after 3 months of use of the probiotic preparation and the content of lactobacilli and bifidobacteria in % of the total amount of bacterial cultures in the sample, the total number of bacterial species in the sample and the number of defecations a day were determined (Table 6B).

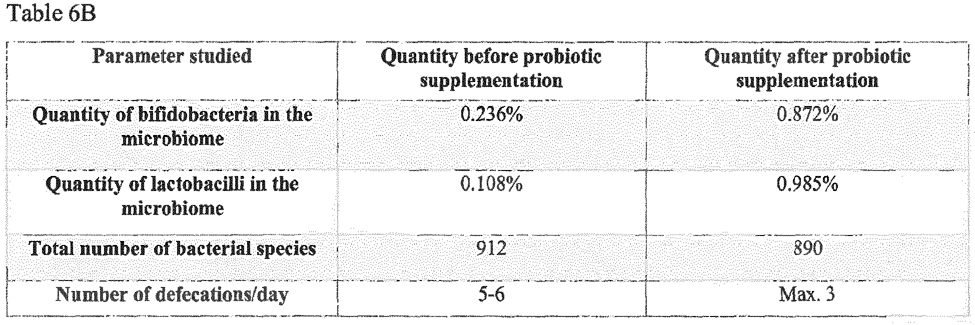

### Example 7

The ratio of lactobacilli and bifidobacteria in the stool sample was determined by molecular biological sequence analysis to a value of 0.025. It was decided on the basis of this ratio that 25 % of the daily dose of probiotics would be formed of bifidobacteria and 75 % of lactobacilli. The overall number of bacterial species in the sample was also determined. Comprehensive molecular biological analysis pointed to a reduced diversity of the client's intestinal microbiome. The client, aged 43, stated frequent illnesses of the respiratory tract and a considerably weakened immunity, with three infections in the previous 6 months, in his medical history. In view of this reduced diversity, the daily dose of probiotic bacteria was set at 45 x 10⁹ CFU. Probiotic bacteria proven by clinical studies or other published output to have a therapeutic effect in relation to illnesses of the respiratory tract^{22;32;33;34} and a positive effect on reinforcing mucosal immunity were used in the probiotic composition on a preferential basis. In view of the given reduced immunity, 40 mg of ascorbic acid and 100 mg of beta glucan from oyster mushrooms were added to the composition. Hydroxypropyl methyl cellulose at an amount of 10% of the total content of the capsule was used as an auxiliary prebiotic substance, promoting the adhesion of probiotic bacteria to the intestinal epithelium, supplemented by lyophilised carrot.

The resultant probiotic composition of a weight of 450 mg, adapted to the form of a granulate in the form of a dry granulation, had the composition given in Table 7A.

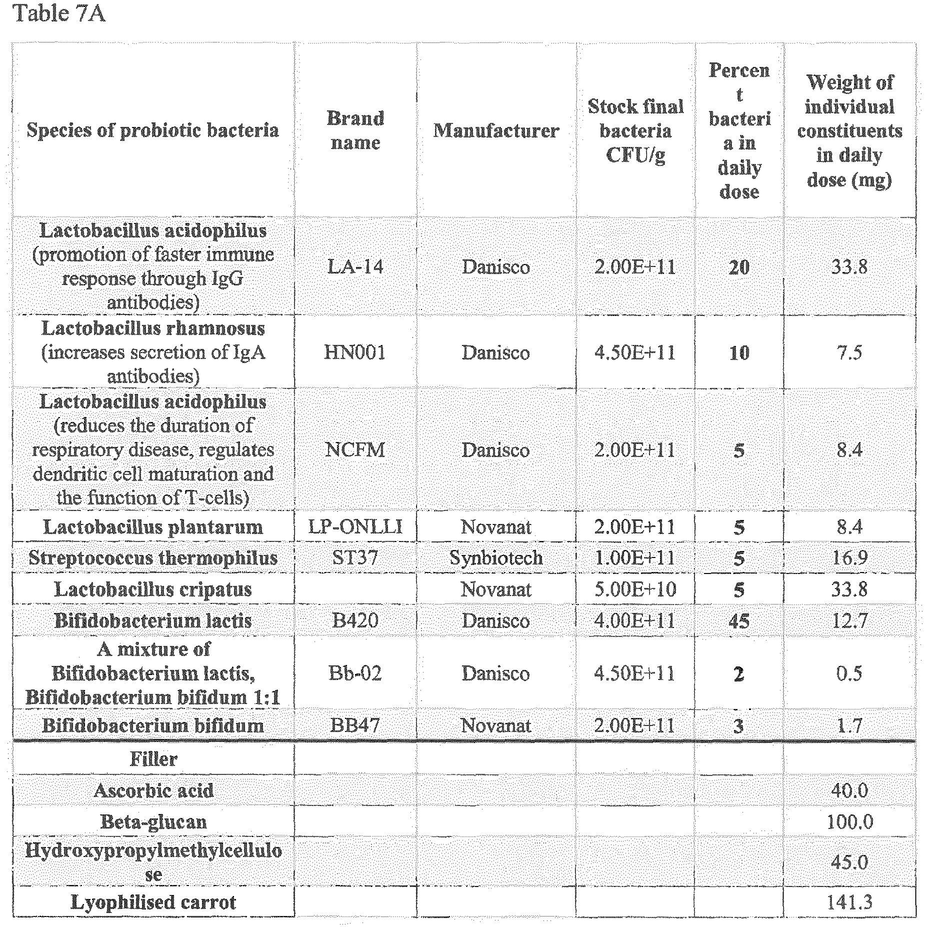

A stool sample was again analysed after 6 months of use of the probiotics and the content of lactobacilli and bifidobacteria in % of the total amount of bacterial cultures in the sample and the total number of bacterial species in the sample were determined, and the number of infections in the last 6 months assessed (Table 7B).

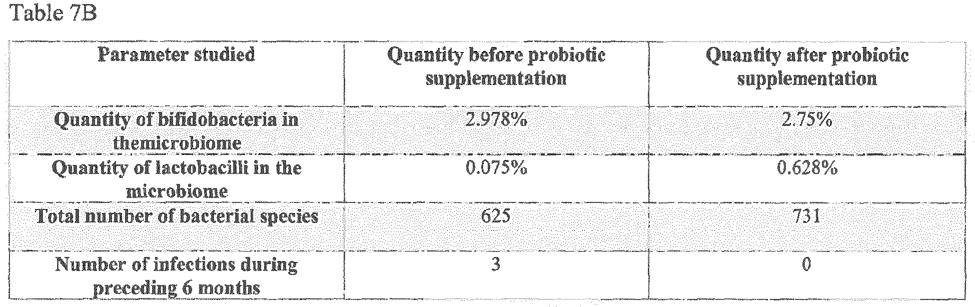

### Example 8

The ratio of lactobacilli and bifidobacteria in the stool sample was determined by Real Time PCR analysis at a value of 0.74. The total number of bacterial species in the sample was also determined. The proportion of probiotic bacteria in the resultant composition was set at 32 % bifidobacteria and 68 % lactobacilli in the daily dose of probiotic bacteria. The client was a woman in the 5^{th} month of pregnancy, for which reason the daily dose was stipulated as 25x10⁹ CFU. Probiotic bacteria that have been proven by clinical studies or other published output to have a positive effect on the developing immune system of the foetus³² or on its overall development and the overall wellbeing of the expectant mother³⁵, including promoting prevention of gestational diabetes³⁶, were used on a preferential basis in the probiotic composition. During the selection of probiotic bacteria, emphasis was also placed on digestive comfort²⁰, support for the client's immune system and support for a healthy vaginal microbiota³⁷. Chicory fructooligosaccharide inulin was used as a prebiotic substance.

The resultant probiotic composition of a weight of 450 mg, adjusted to size 0 gelatine capsules corresponding to the daily dose, had the composition given in Table 8A.

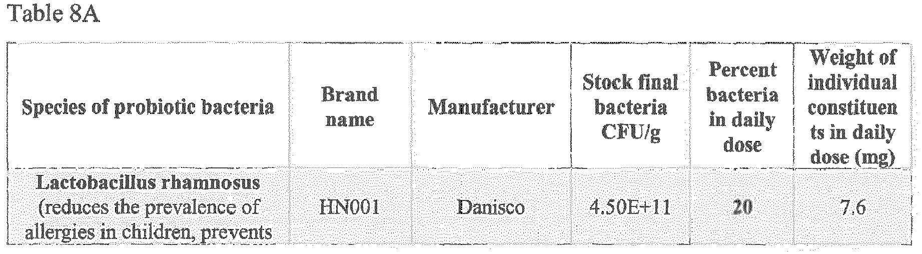

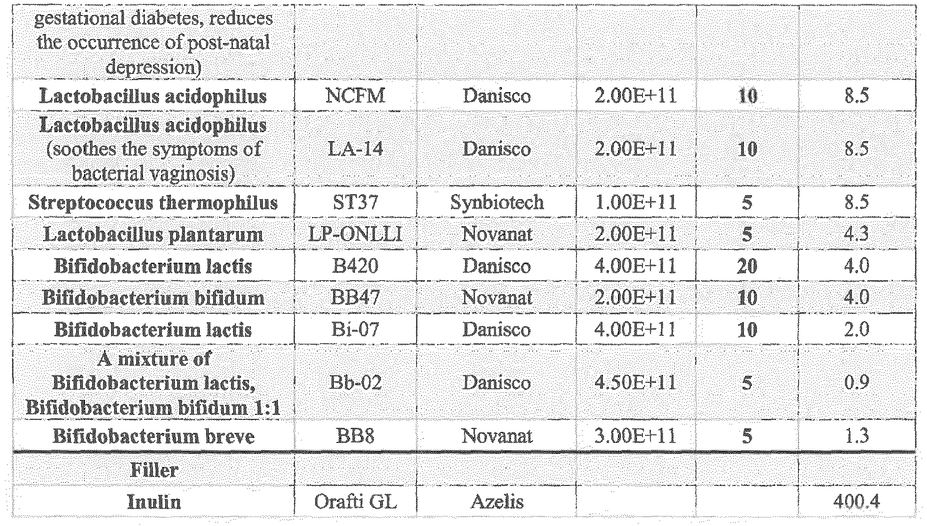

A stool sample was again analysed after 3 months of use of the probiotic preparation and the content of lactobacilli and bifidobacteria in % of the total amount of bacterial cultures in the sample and the total number of bacterial species in the sample were determined (Table 8B).

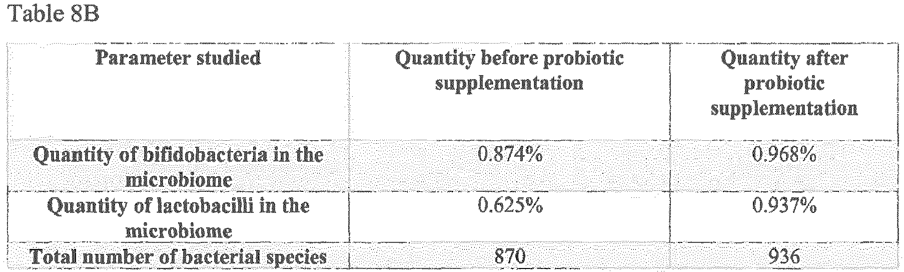

### Example 9

The ratio of lactobacilli and bifidobacteria in the stool sample of a dog, a golden retriever, was determined by molecular biological sequence analysis at a value of 0.035. The total number of bacterial species in the sample was also determined. A decision was made on the basis of this ratio that 25 % of the daily dose of probiotics would be formed of bifidobacteria and 75 % of lactobacilli. Comprehensive molecular biological analysis pointed to the reduced diversity of the intestinal microbiome of the animal. The owner of the dog stated frequent infections of the respiratory tract in the animal's medical history and, according to examination by a vet, the reduced immunity of the animal. In view of this reduced diversity, the daily dose of probiotic bacteria was set at 15 x 10⁹ CFU. Probiotic bacteria proven by clinical studies or other published output to have a therapeutic effect in relation to illnesses of the respiratory tract^{22;32;33;34} and a positive effect on reinforcing the mucosal immunity, regardless of whether this involved studies on humans or animals, were used on a preferential basis in the probiotic composition. In view of this reduced immunity, 100 mg of beta glucan from oats and 40 mg of granulated dried bovine colostrum were added to the composition. Hydroxypropyl methylcellulose at an amount of 10 % of the total content of the capsule, promoting the adhesion of probiotic bacteria to the intestinal epithelium, supplemented with lyophilised carrot and meat aroma, was used as an auxiliary prebiotic substance.

The resultant probiotic composition of a weight of 450 mg, adapted into the form of a powder corresponding to the daily dose, had the composition given in the Table 9A.

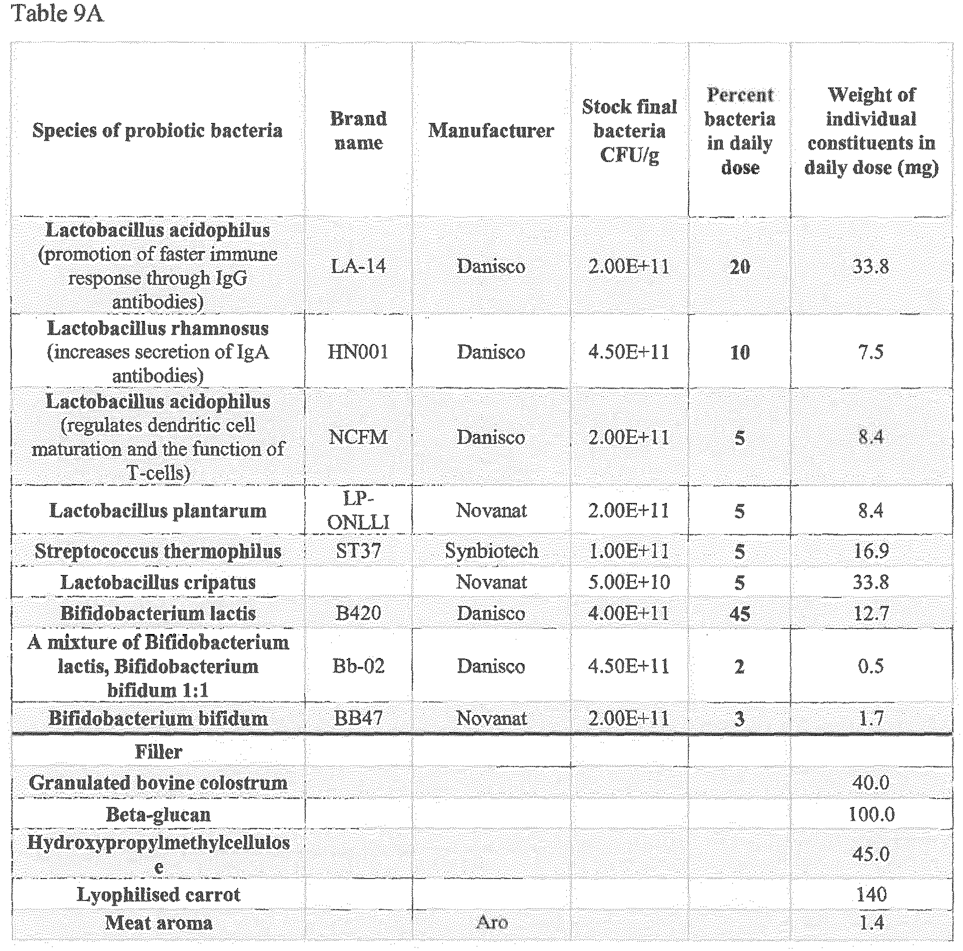

A stool sample was again analysed after 3 months of use of the probiotic preparation and the content of lactobacilli and bifidobacteria in % of the total amount of bacterial cultures in the sample and the total number of bacterial species in the sample were determined (Table 9B).

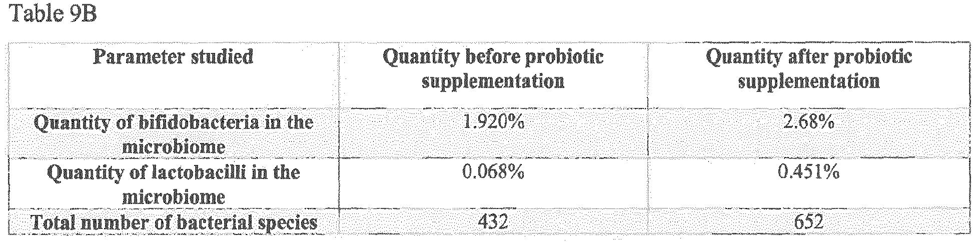

### Example 10

The ratio of lactobacilli and bifidobacteria in the stool sample of a domestic cat was determined by molecular biological analysis with the use of Real Time PCR at a value of 0.016. The total number of bacterial species in the sample was also determined. The proportion of probiotic bacteria in the resultant probiotic composition was proposed as 25 % bifidobacteria and 75 % lactobacilli in the daily dose of probiotic bacteria. In view of the low weight of the animal, the daily dose of probiotics was set at 2 x 10⁹ CFU. An increased frequency of diarrhoea was given in the cat's medical history, for which reason *S. boulardii*³⁸ was used in the probiotic composition. Probiotic species of bacteria targeting increased bacterial diversity in the gut with an emphasis on optimising digestion and reinforcing overall immunity^{21;22;34} were also used in the probiotic composition. A fructooligosaccharide from chicory - inulin - was used as an auxiliary prebiotic substance.

The resultant probiotic composition of a powder mixture of a weight of 1,000 mg corresponding to the daily dose, filled in single-use bags, had the composition given in Table 10A.

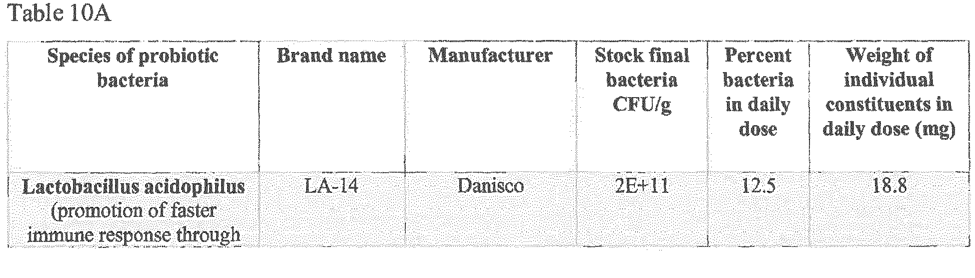

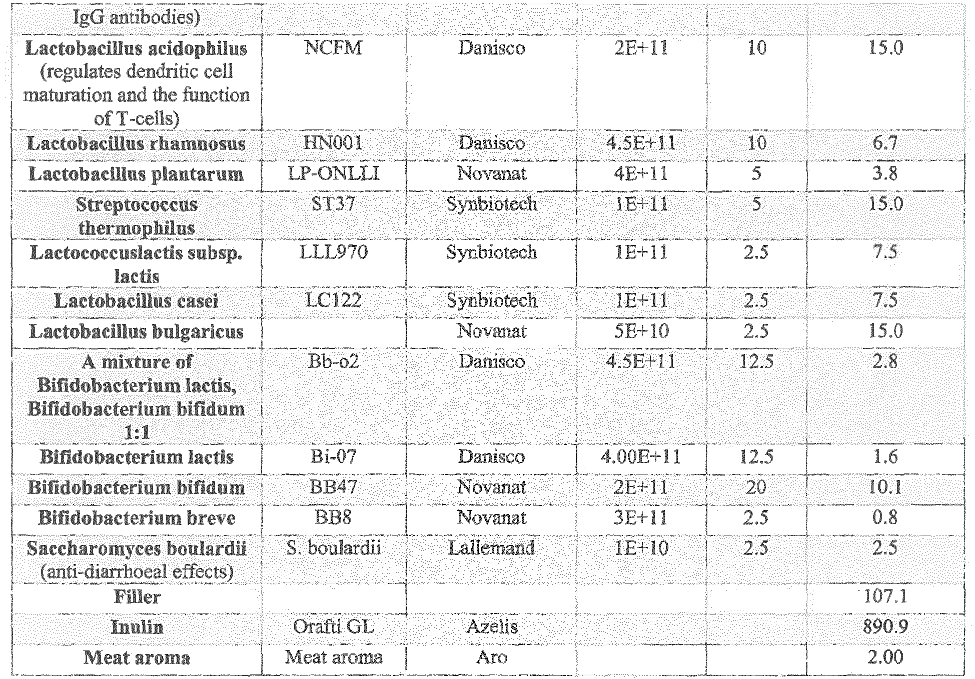

A stool sample was again analysed after 3 months of use of the probiotic preparation and the content of lactobacilli and bifidobacteria in % of the total amount of bacterial cultures in the sample and the total number of bacterial species in the sample were determined (Table 10B).

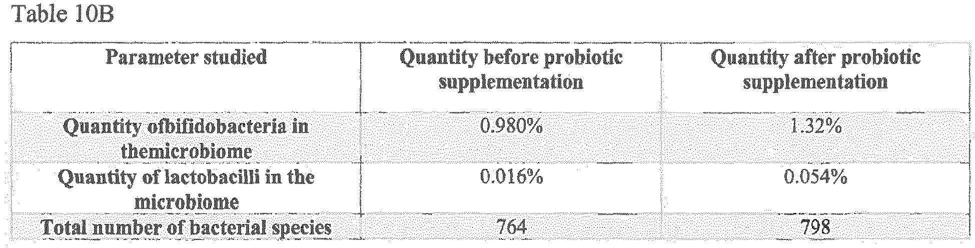

It is clear from the results of the examples given above that 3 months of use of a probiotic preparation led to adjustment of the quantity of lactobacilli and bifidobacteria and the total number of bacterial species in the microbiome and improvement to the monitored parameters in the medical history.

## Claims

1. A method of preparation of an individual probiotic composition for human or veterinary use according to medical history on the basis of analysis of the individual intestinal microbiome, including
a) analysis of the intestinal microbiome of the individual by means of analysis of the individual's stool sample for quantitative determination of bacteria of the genera *Lactobacillus* and *Bifidobacterium* and determination of their ratio and/or determination of the ratio of the bacterial phyla *Firmicutes* and *Bacteroidetes* in the sample
b) preparation of constituent of individual probiotic cultures for a probiotic composition according to the results of analysis of the intestinal microbiome and with the use of probiotic cultures with proven health benefits on at least one of the disorders given in the medical history of the individual according to at least one source in the literature and/or the result of a clinical study
c) an addition of an auxiliary component to the constituents of the individual probiotic culture.

2. The method of preparation of an individual probiotic composition according to claim 1, wherein the analysis of the microbiome in the stool sample is performed by cultivation of the stool sample and/or by the Real Time PCR method and/or by sequencing the stool sample or by any combination of these methods.

3. The probiotic composition prepared by the method of claim 1, wherein the constituent of individual probiotic cultures contains a mixture of at least two different probiotic cultures and an excipient.

4. The probiotic composition according to claim 3, wherein the constituent of individual probiotic cultures contains bacterial genera selected from the group *Lactobacillus, Bifidobacterium, Streptococcus, Bacillus, Saccharomyces, Enterococcus, Lactococcus, Escherichia, Akkermansia, Faecalibacterium, Eubacterium, Leuconostoc* and *Pediococcus* or mixtures of them.

5. The probiotic composition according to claims 3 to 4, containing bacterial cultures at a quantity of 1 x 10³ to 1 x 10¹⁴ CFU per 1 g total weight of the probiotic composition, at least one probiotic culture at a quantity of at least 1 x 10⁵ to 1 x 10¹⁴ CFU per 1g total weight of the probiotic composition, preferentially 50 x 10⁹ CFU to 200 x 10⁹ CFU per 1g total weight of the probiotic composition.

6. The probiotic composition according to claims 3 to 5, containing 1-34 % bifidobacteria and 66-99 % lactobacilli, preferentially 25 % bifidobacteria and 75 % lactobacilli of the total number of probiotic cultures of the constituent of individual probiotic cultures in the probiotic composition if the ratio of lactobacilli and bifidobacteria in the stool sample is lower than 0.099.

7. The probiotic composition according to claims 3 to 5, containing 2-49 % bifidobacteria and 51-98 % lactobacilli, preferentially 35 % bifidobacteria and 65 % lactobacilli of the total number of probiotic cultures of the constituent of individual probiotic cultures in the probiotic composition if the ratio of lactobacilli and bifidobacteria in the stool sample falls within a range of 0.1 to 0.799.

8. The probiotic composition according to claims 3 to 5, containing 20-80 % bifidobacteria and 20-80% lactobacilli, preferentially 50 % bifidobacteria and 50 % lactobacilli of the total number of probiotic cultures of the constituent of individual probiotic cultures in the probiotic composition if the ratio of lactobacilli and bifidobacteria in the stool sample falls within a range of 0.8 to 1.099.

9. The probiotic composition according to claims 3 to 5, containing 20-99 % bifidobacteria and 1-80% lactobacilli, preferentially 75 % bifidobacteria and 25 % lactobacilli of the total number of probiotic cultures of the constituent of individual probiotic cultures in the probiotic composition if the ratio of lactobacilli and bifidobacteria in the stool sample is greater than 1.1.

10. The probiotic composition according to claims 3 to 5 containing probiotic bacteria of the genus *Bifidobacterium* if the ratio of the bacterial phyla *Firmicutes* and *Bacteroidetes* in the stool sample is greater than 1.

11. The probiotic composition according to claims 3 to 5 containing probiotic bacteria of the genus *Lactobacillus* at an amount of 51-100 % of the total number of probiotic cultures of the constituent of individual probiotic cultures in the probiotic composition if the ratio of the bacterial phyla *Firmicutes* and *Bacteroidetes* in the stool sample is lower than 0.8.

12. The probiotic composition according to claims 3 to 5 containing probiotic bacteria of the genus *Lactobacillus* at an amount of 1-99 % and probiotic bacteria of the genus *Bifidobacterium* at an amount of 99-1 % of the total number of probiotic cultures of the constituent of individual probiotic cultures in the probiotic composition if the ratio of the bacterial phyla *Firmicutes* and *Bacteroidetes* in the stool sample is greater than 0.8 and lower than 1.

13. The probiotic composition according to claim 3, where the excipient is selected from a group comprised of inulin, dextrin, maltodextrin, maize starch, potato starch, fructooligosaccharides, galactooligosaccharides, pectin, guar gum, xanthan gum, carrageenan, alginate, fruit extracts, vegetable extracts, cereal extracts, legume extracts, lyophilised fruit, lyophilised vegetables, vitamins, minerals, plant and herb extracts or mixtures of them.

14. A food, pharmaceutical or veterinary preparation containing a probiotic composition according to claims 3 to 13 in the form of a tablet, capsule, powder, gel, suppository, sweet, yoghurt, kefir, solution or granulate.
